(19) Europäisches Patentamt
European Patent Office
Office européen des brevets

(11) Veröffentlichungsnummer: **0 012 261**
**B1**

(12) # EUROPÄISCHE PATENTSCHRIFT

(45) Veröffentlichungstag der Patentschrift: **05.01.83**

(51) Int. Cl.³: **C 07 D 263/52, A 01 N 43/76**

(21) Anmeldenummer: **79104682.4**

(22) Anmeldetag: **24.11.79**

(54) **Substituierte Spiro-Derivate von 3-(3,5-Dichlorphenyl)-oxazolidin-2,4-dionen, Verfahren zu ihrer Herstellung sowie ihre Verwendung als Fungizide.**

(30) Priorität: **07.12.78 DE 2852924**

(43) Veröffentlichungstag der Anmeldung:
**25.06.80 Patentblatt 80/13**

(45) Bekanntmachung des Hinweises auf die Patenterteilung:
**05.01.83 Patentblatt 83/1**

(84) Benannte Vertragsstaaten:
**BE CH DE FR GB IT NL**

(56) Entgegenhaltungen:
**EP - A - 0 003 520**
**GB - A - 982 940**
**US - A - 3 903 090**

**AGRICULTURAL AND BIOLOGICAL CHEMISTRY, Band 35, Nr. 11, November 1971, Tokyo JP A. FUJINAMI et al. "Studies on biological activity of cyclic imide compounds. Part I. Antimicrobial activity of 3-phenyl-oxazolidine-2,4-diones and related compounds". Seiten 1707—1719.**

**Die Akte enthält technische Angaben, die nach dem Eingang der Anmeldung eingereicht wurden und die nicht in dieser Patentschrift enthalten sind.**

(73) Patentinhaber: **BAYER AG**
***Zentralbereich Patente, Marken und Lizenzen***
**D-5090 Leverkusen 1, Bayerwerk (DE)**

(72) Erfinder: **Knops, Hans-Joachim, Dr.**
**Claudiusweg 3**
**D-5600 Wuppertal 1 (DE)**
Erfinder: **Heine, Hans-Georg, Dr.**
**Heckerhof 14**
**D-4150 Krefeld (DE)**
Erfinder: **Draber, Wilfried, Dr.**
**In den Birken 81**
**D-5600 Wuppertal 1 (DE)**
Erfinder: **Brandes, Wilhelm, Dr.**
**Eichendorffstrasse 3**
**D-5623 Leichlingen (DE)**

**EP 0 012 261 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Substituierte Spiro-Derivate von 3-(3,5-Dichlorphenyl)-oxazolidin-2,4-dionen, Verfahren zu ihrer Herstellung sowie ihre Verwendung als Fungizide

Die Vorliegende Erfindung betrifft neue substituierte Spiro-Derivate von 3-(3,5-Dichlorphenyl)-oxazolidin-2,4-dionen, mehrere Verfahren zu ihrer Herstellung sowie ihre Verwendung als Fungizide.

Es ist bereits bekannt geworden, daß Thiuramdisulfide, wie beispielsweise Tetramethyl-thiuramdisulfid, gute fungizide Eigenschaften aufweisen (vergleiche US-Patentschrift 1 972 961). Die Wirkung dieser Stoffklasse ist jedoch in bestimmten Indikationsbereichen, insbesondere bei niedrigen Aufwandmengen und -konzentrationen, nicht immer voll befriedigend.

Es wurden nun als neue Verbindungen die substituierten Spiro-Derivate von 3-(3,5-Dichlorphenyl)-oxazolidin-2,4-dionen der Formel

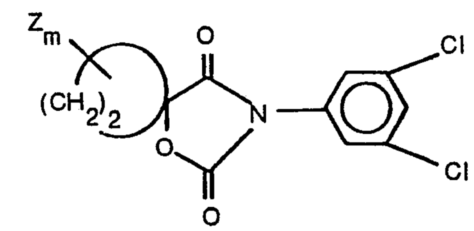

(I)

in welcher

Z für Alkyl mit 1 bis 4 Kohlenstoffatomen oder ankondensiertes Cylcopentan, Cyclolexan, Cyclopenten oder Cyclohexen steht und

m für die ganzen Zahlen 1, 2, 3 oder 4 steht,

gefunden.

Weiterhin wurde gefunden, daß man die substituierten Spiro-Derivate von 3-(3,5-Dichlorphenyl)-oxazolidin-2,4-dionen der Formel (I) erhält, wenn man $\alpha$-Hydroxy-cyclopropylcarbonsäuren bzw. deren Ester der Formel

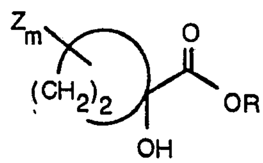

(II)

in welcher

R für Wasserstoff oder Alkyl mit 1 bis 4 Kohlenstoffatomen steht und

Z und m die oben angegebene Bedeutung haben,

a) mit dem Isocyanat der Formel

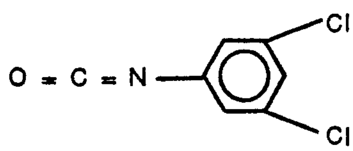

(III)

gegebenenfalls in Gegenwart einer Base und in Gegenwart eines Verdünnungsmittels umsetzt oder

b) mit dem Anilin der Formel

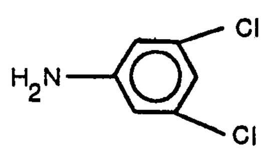

(IV)

in Gegenwart eines Verdünnungsmittels umsetzt und die entstehenden $\alpha$-Hydroxy-cyclopropan-carbonsäureamide der Formel

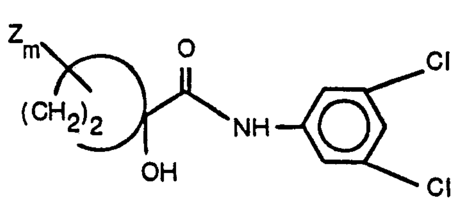

(V)

in welcher

Z und m die oben angegebene Bedeutung haben, mit Phosgen in Gegenwart einer Base cyclisiert.

Die neuen substituierten Spiro-Derivate von 3-(3,5-Dichlorphenyl)-oxazolidin-2,4-dionen der Formel (I) weisen gute fungizide Eigenschaften auf.

Ueberraschend zeigen die erfindungsgemäßen substituierten Spiro-Derivate von 3 -(3,5-Dichlorphenyl)-oxazolidin-2,4-dionen eine erheblich höhere fungizide Wirkung, insbesondere gegen Botrytis-Arten, als die aus dem Stand der Technik bekannte Verbindung Tetramethylthiuramdisulfid, welches ein anerkannt gutes Mittel gleicher Wirkungsrichtung ist. Die erfindungsgemäßen Wirkstoffe stellen somit eine Bereicherung der Tecknik dar.

Verwendet man zur Herstellung beispielsweise 1-Hydroxy-2-methyl-cyclopropan-1-carbonsäure und 3,5-Dichlorphenylisocyanat als Ausgangsstoffe, so kann der Reaktionsablauf durch das folgende Formelschema wiedergegeben werden (Verfahren a):

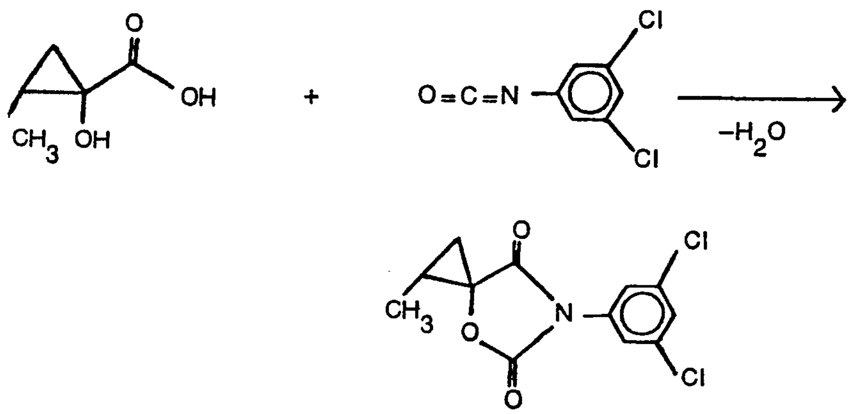


Verwendet man beispielsweise 1-Hydroxy-2-methyl-cyclopropan-1-carbonsäure, 3,5-Dichloranilin und Phosgen als Ausgangsstoffe, so kann der Reaktionsablauf durch das folgende Formelschema wiedergegeben werden (Verfahren b):

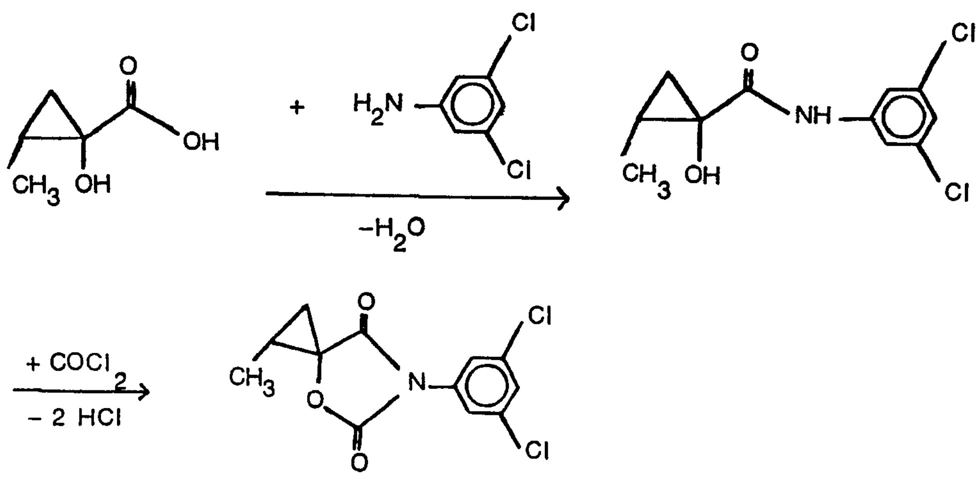


Die als Ausgangsstoffe zu verwendenden $\alpha$-Hydroxy-cyclopropylcarbonsäuren(ester) sind durch die Formel (II) allgemein definiert. In dieser Formel steht *R* vorzugsweise für Wasserstoff, Methyl oder Ethyl.

Die Ausgangsstoffe der Formel (II) sind weitgehend bekannt (vergleiche z.B. Liebigs Ann. Chem. *1976* 463; J. Chem. Soc. *1938,* 1211 und DE—OS 21 28 327). Als Beispiele seien genannt:
1-Hydroxy-2-methyl-cyclopropan-1-carbonsäure; -methyl- und -ethylester;
1-Hydroxy-2-ethyl-cyclopropan-1-carbonsäure; -methyl- und -ethylester;
1-Hydroxy-2-isopropyl-cyclopropan-1-carbonsäure; -methyl- und -ethylester;
1-Hydroxy-2-tert.-butyl-cyclopropan-1-carbonsäure; -methyl- und -ethylester;
1-Hydroxy-2,3-dimethyl-cyclopropan-1-carbonsäure; -methyl- und -ethylester;
1-Hydroxy-2,2,3,3-tertamethyl-cyclopropan-1-carbonsäure; -methyl- und -ethylester;

Die Ausgangsstoffe der Formeln (III) und (IV) sind allgemein bekannte Verbindungen der organischen Chemie.

Für die erfindungsgemäße Umsetzung gemäß Verfahrensvariante (a) kommen als Verdünnungsmittel vorzugsweise inerte organische Lösungsmittel infrage. Hierzu gehören vorzugsweise aromatische Kohlenwasserstoffe, wie beispielsweise Benzol, Toluol, Xyol oder 1,2-Dichlorbenzol, sowie aliphatische, halogenierte Kohlenwasserstoffe, wie beispielsweise Methylenchlorid, Chloroform oder Tetrachlorkohlenstoff.

Wird die erfindungsgemäße Umsetzung gemäß Verfahrensvariante (a) in Gegenwart einer Base durchgeführt, so können alle üblicherweise verwendbaren organischen und anorganischen Basen eingesetzt werden. Hierzu gehören vorzugsweise tertiäre Amine, wie beispielsweise Triäthylamin oder Pyridin sowie Alkoholate, wie beispielsweise Kalium- oder Natrium-tert.-butylat.

Die Reaktionstemperaturen können bei der erfindungsgemäßen Verfahrensvariante (a) in einem größeren Bereich variiert werden. Im allgemeinen arbeitet man zwischen 20 und 150°C, vorzugsweise bei der Siedetemperatur des jeweiligen Lösungsmittels.

Bei der Durchführung der erfindungsgemäßen Verfahrensvariante (a) arbeitet man vorzugsweise in molaren Mengen. Bei Verwendung einer Base wird diese in äquimolarer Menge eingesetzt, wenn man $\alpha$-Hydroxycyclopropancarbonsäuren als Ausgangsstoffe verwendet und lediglich in katalytischer Menge, wenn $\alpha$-Hydroxycyclopropancarbonsäureester eingesetzt werden. Zur Isolierung der Verbindungen der Formel (I) wird das Lösungsmittel abdestilliert und der Rückstand nach üblichen Methoden aufgearbeitet.

Für die erfindungsgemäße Umsetzung gemäß Verfahrensvariante (b) kommen als Verdünnungsmittel vorzugsweise inerte organische Lösungsmittel infrage. Hierzu gehören vorzugsweise die bei der Verfahrensvariante (a) bereits genannten Solventien.

Als Basen kommen für die erfindungsgemäße Umsetzung gemäß Verfahrensvariante (b) vorzugsweise die bei der Variante (a) bereits genannten Reagenzien infrage.

Die Reaktionstemperaturen können bei der erfindungsgemäßen Verfahrensvariante (b) in einem größeren Bereich variiert werden. Im allgemeinen arbeitet man zwischen 20 und 150°C, vorzugsweise bei der Siedetemperatur des verwendeten Lösungsmittels.

Bei der Durchführung der erfindungsgemäßen Verfahrensvariante (b) arbeitet man vorzugsweise in molaren Mengen. Die als Zwischenprodukt auftretenden $\alpha$-Hydroxy-cyclopropancarbonsäureamide der Formel (V) können ohne Isolierung direkt umgesetzt werden. Zur Isolierung der Verbindungen der Formel (I) wird das Lösungsmittel abdestilliert und der Rückstand nach üblichen Methoden aufgearbeitet.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Botrytis-Arten, wie gegen den Erreger des Grauschimmels (Botrytis cinerea) verwendet werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Keiselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischen Material wie Sägemahl, Kokosnußschalen, Maiskolben und Tabakstengel; als Emulgier- und/oder schaumerzeugende Mittel kommen in Frage; z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fugiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramme Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Beispiel A

Botrytis-Test (Bohnen)/protektiv
Lösungsmittel: 4,7 Gewichtsteile Aceton
Dispergiermittel: 0,3 Gewichtsteile Alkyl-arylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.

Die erhaltenen Boniturwerte werden auf Prozent Befall umgerechnet. 0% bedeutet keinen Befall, 100% bedeutet, daß der Befallsfleck vollständig ausgebildet ist.

Bei diesem Test zeigen z.B. die folgenden Verbindungen eine sehr gute Wirkung, die derjenigen der aus dem Stand der Technik bekannten Verbindungen überlegen ist.

Verbindung gemäß Herstellungsbeispiel 1.

## Herstellungsbeispiele

### Beispiel 1

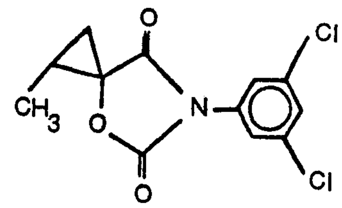

(Verfahren a)

5,8 g (0,05 Mol) 1-Hydroxy-2-methyl-cyclopropan-1-carbonsäure und 5,05 g (0,05 Mol) Triethylamin werden in 200 ml 1,2-Dichlorbenzol bei ca. 100°C gelöst. Bei dieser Temperatur tropft man unter Rühren eine Lösung von 9,4 g (0,05 Mol) 3,5-Dichlorphenylisocyanat in 50 ml 1,2-Dichlorbenzol hinzu und erhitzt danach 1 Stunde unter Rückfluß am Wasserabscheider. Nach beendeter Wasserabscheidung läßt man abkühlen und engt durch Abdestillation des Lösungsmittels im Vakuum ein. Der noch warme Rückstand wird mit 100 ml heißem Ethanol versetzt und gut durchgemischt. Während des Abkühlens scheiden sich Kristalle ab, die abgesaugt, mit wenig Ethanol gewaschen und getrocknet werden. Man erhält 7,2 g (50% der Theorie) 1-Oxa-3-aza spiro[4,2]-heptan-3-(3,5-dichlorphenyl)-6-methyl-2,4-dion vom Schmelzpunkt 125—27°C.

In analoger Weise entsprechend Verfahren (a) oder gemäß Verfahren (b) werden die folgenden Verbindungen der allgemeinen Formel

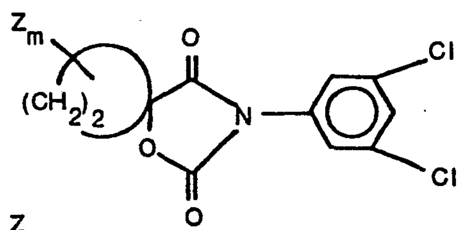 (I)

erhalten:

| Bsp. Nr. | 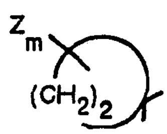 | Schmelzpunkt (°C) |
|---|---|---|
| 2 | 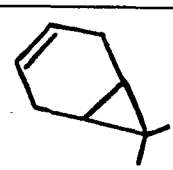 | 172–75 |
| 3 | 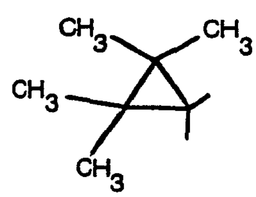 | 154 |
| 4 | 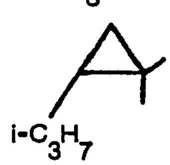 | 131 |
| 5 | 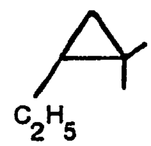 | 103 |
| 6 | 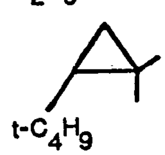 | 133-34 |

**Patentansprüche**

1. Substituierte Spiro-Derivate von 3-(3,5-Dihalogenphenyl)-oxazolidin-2,4-dionen der allgemeinen Formel

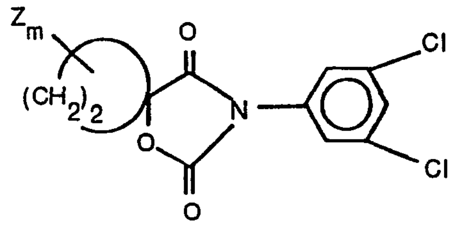

(I)

in welcher
Z für Alkyl mit 1 bis 4 Kohlenstoffatomen oder ankondensiertes Cylcopentan, Cyclolexan, Cyclopenten oder Cyclohexen steht und
m für die ganzen Zahlen 1, 2, 3 oder 4 steht,

2. Verfahren zur Herstellung von substituierten Spiro-Derivaten von 3-(3,5-Dihalogenphenyl)-oxazolidin-2,4-dionen der Formel (I) in Anspruch 1, dadurch gekennzeichnet, daß man $\alpha$-Hydroxy-cycloalkylcarbonsäuren bzw. deren Ester der Formel

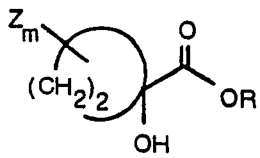

(II)

in welcher
R für Wasserstoff oder Alkyl mit 1 bis 4 Kohlenstoffatomen steht und
Z und m die in Anspruch 1 angegebene Bedeutung haben,
a) mit einem Isocyanate der Formel

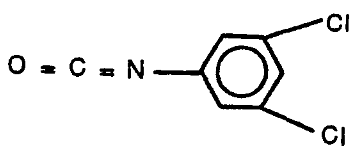

(III)

gegebenenfalls in Gegenwart einer Base und in Gegenwart eines Verdünnungsmittels umsetzt oder
b) mit einem Anilin der Formel

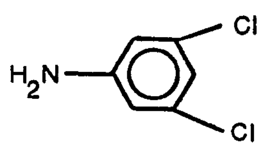

(IV)

in Gegenwart eines Verdünnungsmittels umsetzt und die entstehenden $\alpha$-Hydroxy-cyclopropancarbonsäureamide der Formel

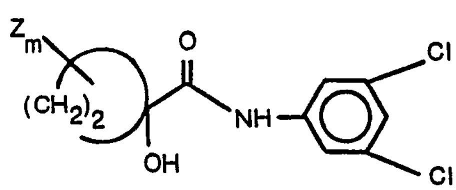

(V)

in welcher
Z und m die in Anspruch 1 angegebene Bedeutung haben,
mit Phosgen in Gegenwart einer Base cyclisiert.

3. Fungizide Mittel, gekennzeichnet durch einen Gehalt an mindestens einem substituierten Spiro-Derivat von 3-(3,5-Dihalogenphenyl)-oxazolidin-2,4-dionen gemäß Anspruch 1.

4. Verwendung von substituierten Spiro-Derivaten von 3-(3,5-Dihalogenphenyl)-oxazolidin-2,4-dionen gemäß Anspruch 1 zur Bekämpfung von Pilzen.

5. Verfahren zur Herstellung von fungiziden Mitteln, dadurch gekennzeichnet, daß man substituierte Spiro-Derivate von 3-(3,5-Dihalogenphenyl)-oxazolidin-2,4-dionen gemäß Anspruch 1 mit Streckmitteln und/oder oberflächenaktiven Mitteln vermischt.

**Revendications**

1. Spiro-dérivés substitués de 3-(3,5-dihalogénophényl)-oxazolidine-2,4-diones de formule générale:

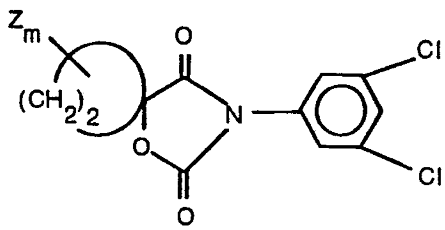

(I)

dans laquelle
Z représente un groupe alkyle contenant 1 à 4 atomes de carbone ou un groupe cyclohexène, cyclopentène, cyclohexane ou cyclopentane condensé, et
m représente les nombres entiers 1, 2, 3 ou 4.

2. Procédé de préparation de spiro-dérivés substitués de 3-(3,5-dihalogénophényl)-oxazolidine-2,4-diones de formule (I) suivant la revendication 1, caractérisé en ce qu'on fait réagir des acides $\alpha$-hydroxy-cycloalkyl-carboxyliques ou leurs esters de formule:

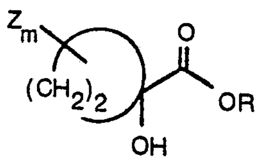

(II)

dans laquelle
R représente un atome d'hydrogène ou un groupe alkyle contenant 1 à 4 atomes de carbone, et
Z et m ont les significations indiquées dans la revendication 1,
a) avec un isocyanate de formule:

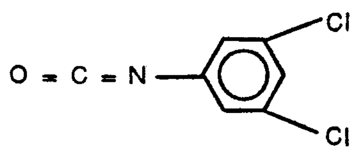

(III)

éventuellement en présence d'une base et en présence d'un diluant, ou
b) avec une aniline de formule:

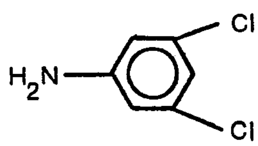

(IV)

en présence d'un diluant et en cyclisant les amides d'acides $\alpha$-hydroxy-cyclo-alcane-carboxyliques obtenus de formule:

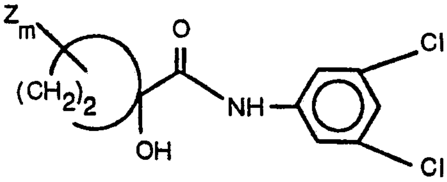

(V)

dans laquelle
Z et m ont les significations indiquées dans la revendication 1, avec du phosgène en présence d'une base.

3. Agents fongicides, caractérisés en ce qu'ils contiennent au moins un spiro-dérivé substitué de 3-(3,5-dihalogénophényl)-oxazolidine-2,4-diones suivant la revendication 1.

4. Utilisation de spiro-dérivés substitués de 3-(3,5-dihalogénophényl)-oxazolidine-2,4-diones suivant la revendication 1 pour combattre les champignons.

5. Procédé de préparation d'agents fongicides, caractérisé en ce qu'on mélange des spiro-dérivés substitués de 3-(3,5-dihalogénophényl)-oxazolidine-2,4-diones suivant la revendication 1 avec des diluants et/ou des agents tensio-actifs.

## Claims

1. Substituted spiro-derivatives of 3-(3,5-dihalogenophenyl)-oxazolidine-2,4-diones of the general formula

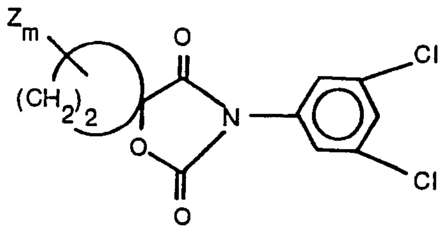

(I)

in which Z represents alkyl with 1 to 4 carbon atoms or fused cyclopentane, cyclohexane, cyclopentene or cyclohexene and
m represents the integers 1, 2, 3 or 4.

2. Process for the preparation of substituted spiro-derivatives of 3-(3,5-dihalogenophenyl)-oxazolidine-2,4-diones of the formula (I) in Claim 1, characterised in that $\alpha$-hydroxycycloalkylcarboxylic acids or their esters, of the formula

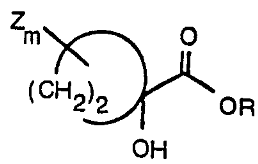

(II)

in which
R represents hydrogen or alkyl with 1 to 4 carbon atoms and Z and m have the meaning indicated in Claim 1,
a) are reacted with a isocyanate of the formula

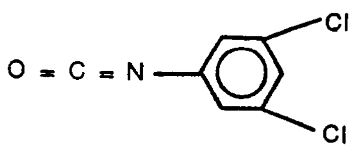

(III)

if appropriate in the presence of a base and in the presence of a diluent or
b) are reacted with an aniline of the formula

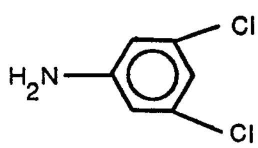

(IV)

in the presence of a diluent and the resulting $\alpha$-hydroxy-cycloalkanecarboxylic acid amides of the formula

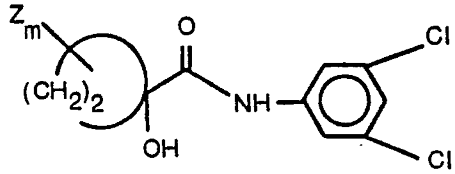

(V)

in which
Z and m have the meaning indicated in Claim 1, are cyclised with phosgene in the presence of a base.

3. Fungicidal agents, characterised in that they contain at least one substituted spiro-derivative of 3-(3,5-dihalogenophenyl)-oxazolidine-2,4-diones according to Claim 1.

4. Use of substituted spiro-derivatives of 3-(3,5-dihalogenophenyl)-oxazolidine-2,4-diones according to Claim 1 for combating fungi.

5. Process for the preparation of fungicidal agents characterised in that substituted spiro-derivatives of 3-(3,5-dihalogenophenyl)-oxazolidine-2,4-diones according to Claim 1 are mixed with extenders and/or surface-active agents.